# FASCICULE DE BREVET EUROPEEN

(11) **EP 1 139 933 B1**
(45) Date de publication et mention de la délivrance du brevet: **23.07.2008**
(21) Numéro de dépôt: 99958283.6
(22) Date de dépôt: 09.12.1999
(51) Int. Cl.: A61F 2/44

(54) **PROTHESE DE DISQUE INTERVERTEBRAL A CORPS COMPRESSIBLE**
BANDSCHEIBENPROTHESE MIT KOMPRESSIBLEM KÖRPER
INTERVERTEBRAL DISC PROSTHESIS WITH COMPRESSIBLE BODY

(30) Priorité: 11.12.1998 FR 9815670
(43) Date de publication de la demande: 10.10.2001
(73) Titulaire: Stryker Spine, 33610 Cestas (FR)
(72) Inventeur: Gauchet, Fabien, 60800 Duvy (FR)
(74) Mandataire: Le Forestier, Eric
(86) Numéro de dépôt international: PCT/FR1999/003071
(87) Numéro de publication internationale: WO 2000/035383

(56) Documents cités:
- EP-A- 0 277 282
- DE-A- 2 263 842
- DE-U- 9 000 094
- FR-A- 2 723 841

## Description

L'invention concerne les prothèses de disque intervertébral.

On connaît d'après le document EP-0 356 112 une prothèse de disque intervertébral comportant deux plateaux généralement plans et un corps en élastomère interposé entre les plateaux et fixés à ceux-ci par ses faces planes d'extrémité. Le comportement mécanique de cette prothèse est assez proche de celui d'un disque intervertébral naturel sain, notamment lorsque le corps est comprimé entre les deux plateaux.

Le document DE-90 00 094 U divulgue une prothèse conforme au préambule de la revendication 1.

Un but de l'invention est de fournir une prothèse de disque d'un type différent et permettant d'approcher davantage les propriétés mécaniques d'un disque intervertébral naturel sain.

En vue de la réalisation de ce but, on prévoit selon l'invention une prothèse selon la revendication 1.

Ainsi, lorsque la sollicitation du corps en compression augmente à partir d'une valeur modérée ou nulle, la hauteur du corps, mesurée de l'un à l'autre des plateaux, se réduit de façon relativement rapide. Ensuite, à mesure qu'augmente la zone de contact, la réaction du corps se fait plus importante et il faut exercer une sollicitation comparativement plus importante pour réduire la hauteur d'une valeur équivalente. En d'autres termes, pour les valeurs de compression les plus basses, la réaction mécanique du corps lors de la compression varie très peu en fonction du changement de hauteur. Par conséquent, la courbe représentant la sollicitation exercée en fonction de la variation de hauteur est peu inclinée par rapport à l'horizontale pour de faibles valeurs de compression. On fournit ainsi peu d'efforts en début de course. Cette propriété est également celle d'un disque naturel sain. On peut donc adapter le comportement du corps non seulement par le choix du matériau mais en outre par la forme de la ou des faces d'extrémité à zone de contact variable pour approcher au plus près les propriétés mécaniques d'un disque intervertébral naturel sain.

Les revendications 2-36 présentent des modes de réalisation.

Avantageusement, la zone de contact est définie par une face du plateau et une face de l'extrémité du corps, les deux faces étant courbes dans au moins une direction commune et étant respectivement concave et convexe, la face concave ayant au moins un rayon de courbure supérieur à un rayon de courbure correspondant de la face convexe.

Ainsi, cette configuration permet de mettre en oeuvre les variations de réaction mécanique telles que précitées. En outre, lorsque le corps est libre de se déplacer latéralement par rapport au plateau, comme on le verra plus loin, cette configuration assure le centrage relatif des deux faces. Par exemple, après que les deux faces ont été décalées, ces courbures permettent qu'elles se recentrent automatiquement.

Avantageusement, le corps présente au moins une extrémité en contact avec l'un des plateaux et libre de se déplacer par rapport au plateau suivant une direction parallèle au plateau.

Ainsi, cet agencement réduit le risque de voir apparaître une sollicitation trop importante entre les deux vertèbres suivant une direction perpendiculaire à la direction longitudinale du rachis, c'est-à-dire en cisaillement.

Avantageusement, le corps présente une extrémité logée dans un renfoncement de l'un des plateaux apte à former une butée latérale pour cette extrémité.

Ainsi, on peut limiter les déplacements latéraux du corps par rapport au plateau, voire les interdire.

Avantageusement, le corps comprend un matériau viscoélastique, notamment du silicone.

Un tel matériau permet de donner une forme en hystérésis à la courbe représentant la sollicitation du corps en compression par rapport à sa variation de hauteur. Cette courbe étant par ailleurs celle d'un disque naturel sain, on approche donc encore mieux ses propriétés mécaniques.

Avantageusement, la prothèse comprend un fluide interposé entre les plateaux.

Ainsi, l'adjonction d'un fluide accroît la forme en hystérésis, notamment lorsque le fluide est compressible tel qu'un gaz ou un mélange d'un liquide et d'un gaz partiellement soluble dans le liquide.

Avantageusement, le fluide est en contact avec les plateaux.

Avantageusement, le fluide s'étend en périphérie du corps.

Avantageusement, la prothèse comporte une enceinte renfermant le fluide et agencée de sorte qu'elle a une superficie de section transversale parallèlement aux plateaux sensiblement invariable lorsque varie une sollicitation des plateaux l'un vers l'autre.

Avantageusement, la prothèse est destinée à la zone lombaire du rachis.

D'autres caractéristiques et avantages de l'invention apparaîtront encore dans la description suivante d'un mode préféré de réalisation et de deux variantes donnés à titre d'exemples non limitatifs. Aux dessins annexés :
- la figure 1 est une vue en perspective d'une prothèse selon l'invention ;
- la figure 2 est une vue en coupe axiale selon le plan II-II de la prothèse de la figure 1 ;
- la figure 3 est une vue à échelle agrandie d'un détail D de la figure 2 ;
- la figure 4 est une courbe indiquant la force de compression F exercée par les deux plateaux sur le coussin en fonction de la variation de la distance les séparant ;
- la figure 5 est une vue en coupe d'un détail d'une variante de réalisation de la prothèse ; et
- la figure 6 est une vue simplifiée analogue à la figure 2 montrant une deuxième variante de réalisation.

La prothèse de disque intervertébral 2 selon l'invention est ici particulièrement destinée à la zone lombaire de la colonne vertébrale du corps humain. Elle comporte deux plateaux plats 4 ayant une forme générale en haricot à hile postérieur en vue en plan. Chaque plateau 4 comporte une plaque circulaire centrale 6 et une couronne 8 s'étendant en périphérie de la plaque dans le plan de celle-ci. Au repos, les deux plateaux 4 s'étendent parallèlement l'un à l'autre, à distance et en regard l'un de l'autre avec leurs contours en coïncidence. Sur chaque plateau 4, la couronne 8 et la plaque 6 présentent chacune une gorge 27 pour la réception d'un joint 31.

La prothèse de disque 2 comporte un coussin ou partie intermédiaire 10 interposé entre les deux plateaux 4. Le coussin comporte un corps solide compressible 12, ici en matériau viscoélastique, par exemple en silicone. Ce corps a une dureté shore-A avantageusement comprise entre 60 et 100, et ici d'environ 80. Le corps 12 a une forme de révolution autour de son axe principal 14. Il présente une face latérale cylindrique 16 et deux faces d'extrémités axiales 18 généralement perpendiculaires à l'axe 14 et de forme légèrement sphérique convexe. Chaque face 18 présente donc deux courbures identiques dans des plans perpendiculaires entre eux. Le corps 12 est disposé coaxialement avec les plaques 6. Chaque plaque 6 présente une face centrale interne plane 20 perpendiculaire à l'axe 14 et en contact avec une des extrémités axiales 18 respectives du corps 12. Ainsi, la face sphérique convexe 18 du corps est en appui sur la face plane 20 du plateau. Le corps 12 est en appui sans ancrage sur chacun des plateaux 4 de sorte qu'il est mobile par rapport à chacun de ces plateaux suivant une direction parallèle aux plateaux, c'est-à-dire perpendiculaire à l'axe principal 14. On évite ainsi la transmission de contraintes latérales de l'une à l'autre des vertèbres.

Le coussin 10 comporte en outre un soufflet 22. Le soufflet entoure le corps 14 coaxialement à celui-ci et à distance de celui-ci. Il a une forme symétrique de révolution autour de l'axe 14. Sa paroi présente de profil des ondulations 24 permettant de faire varier la longueur du soufflet 22 suivant la direction axiale 14, sans que varie sensiblement la superficie de sa section transversalement à l'axe 14. En l'espèce, ce soufflet, de même que les plateaux 4, est réalisé en titane ou alliage de titane, de sorte qu'il présente une certaine rigidité axiale et forme un ressort de compression. Il peut également être déformé suivant une direction perpendiculaire à l'axe 14 ou subir une torsion autour de l'axe 14 ou d'un axe quelconque perpendiculaire à celui-ci.

Le soufflet 22 présente à ses deux extrémités axiales des bords collés à des bords respectifs des plaques 6 s'étendant en saillie de la face interne 20. Le collage est réalisé de façon étanche de sorte que le soufflet 22 définit avec les deux plaques 6 une enceinte étanche à volume variable s'étendant autour du corps 12. Cette enceinte renferme un fluide, en l'espèce un gaz qui est ici de l'air. Les ondulations 24 les plus proches du corps 12 s'étendent à distance de celui-ci pour permettre une libre circulation du gaz de l'une à l'autre des plaques 6.

Le soufflet 22 présente en l'espèce dix convolutions, soit huit crêtes externes en plus des deux crêtes de fixation aux plateaux. Il a ici un diamètre externe d'environ 30 mm et un diamètre interne d'environ 17 mm. Sa hauteur, lorsque la prothèse est hors charge, vaut 10 mm. La paroi du soufflet peut être réalisée au moyen d'une, deux ou trois feuilles chacune de 0,1 mm d'épaisseur et dont la somme des épaisseurs forme l'épaisseur de la paroi. Le soufflet a ici en propre une raideur d'environ 1,6 N/mm.

Chaque couronne 8 comporte deux pattes 25 s'étendant en saillie d'une face externe du plateau 4 perpendiculairement au plan du plateau. Chaque patte 25 présente un orifice 27 la traversant de part en part en direction du centre de la plaque et, sur une face de la patte 25 opposée au plateau 4, une empreinte de forme sphérique. Les orifices 27 permettent la réception d'une vis à os 26 ayant une tête 28 dont une face inférieure a une forme sphérique mâle coopérant avec l'empreinte femelle de la patte 25 pour permettre une libre orientation de la vis 26 par rapport à la patte associée.

Pour réaliser un ancrage à court terme de la prothèse de disque 2 dans la colonne, on pourra ancrer les vis 26 dans le spondyle des vertèbres adjacentes au disque à remplacer.

Toutefois, on pourra prévoir un ancrage dit à long terme où, en outre, les surfaces des plateaux 4 en contact avec les vertèbres adjacentes sont recouvertes d'hydroxyapatite, ou de toute autre substance connue en soi pouvant stimuler la croissance osseuse. Avant recouvrement, lesdites surfaces pourront être traitées pour obtenir un état de surface plus ou moins poreux, présentant des points d'ancrage pour le tissu osseux, pour assurer une meilleure interface avec ledit tissu osseux.

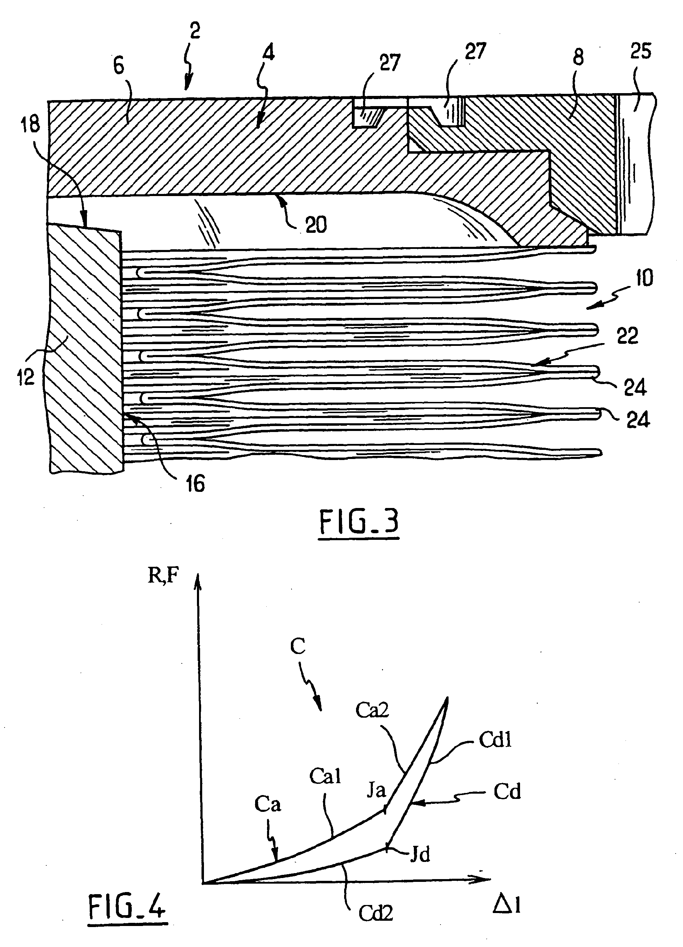

On a représenté en figure 4 l'allure de la courbe C indiquant l'intensité d'un effort de compression F exercé sur le coussin 10 (c'est-à-dire sur les deux plateaux 4) en faisant abstraction de leur déformabilité, quasi nulle, suivant la direction axiale 14, en fonction de la variation de la longueur 1 du coussin suivant la direction axiale 14 (ou encore de la distance entre les deux plateaux). Cette courbe représente également la réaction mécanique R du coussin 10 dans les mêmes conditions. Cette courbe C n'est pas linéaire. De plus, elle présente une forme en hystérésis : la courbe Ca indiquant l'augmentation de la compression F à partir de l'origine zéro étant distincte de celle Cd indiquant la diminution de la compression F jusqu'à l'origine, et s'étendant tout entière au-dessus de cette dernière. Cette forme en hystérésis prononcée est due principalement au matériau viscoélastique du corps et subsidiairement à l'association dans le coussin 10 du corps 12 et du fluide.

En outre, la courbe Ca, relative à l'augmentation de la force de compression F, présente à partir de l'origine O une portion Ca1 à faible pente, puis une portion Ca2 à pente plus forte. La courbe Cd illustrant la diminution de la compression F présente pour les valeurs les plus élevées de la force F une portion Cd1 de forte pente, puis pour les valeurs les plus basses de la force F une portion Cd2 de pente plus faible. La présence d'une portion de faible pente au voisinage de l'origine pour les courbes Ca et Cd est due principalement à la conformation des faces de contact 18, 20 du corps 12 et des plateaux 4, qui entraîne que la superficie de la zone de contact mutuel entre chaque plateau et le corps, généralement en forme de disque, augmente lorsqu'on augmente la force F. Cette augmentation se produit jusqu'à atteindre la superficie maximale de la zone de contact, lorsque toute la face 18 touche le plateau 4.

Les points de raccordement Ja et Jd forment respectivement la jonction entre les courbes Ca1 et Ca2, et Cd1 et Cd2. Sur la courbe Ca, le point Ja correspond à l'effort F pour lequel les surfaces maximales de contact entre les plateaux et le corps sont atteintes. De même, sur la courbe Cd, le point Jd correspond à l'effort pour lequel ces surfaces cessent d'être maximales.

La prothèse pourra être configurée de sorte que le point Ja corresponde à une valeur de Δl située entre 25% et 75% de la variation maximale de longueur envisagée pour la prothèse en utilisation.

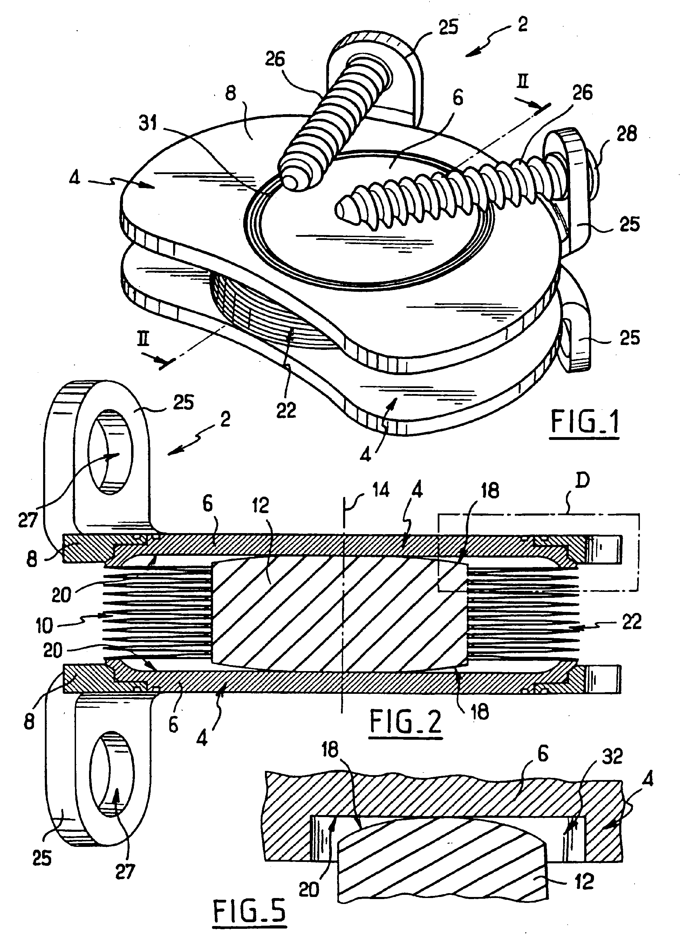

En référence à la figure 5, on pourra prévoir dans une variante de réalisation (présentant par ailleurs les autres caractéristiques de la prothèse de la figure 1) que la face 20 de chaque plateau 4 en regard du corps 12 présente un renfoncement 32, ici en « U », formant butée latérale, dans lequel vient se loger l'extrémité axiale 18 correspondante du corps. On limite ainsi à une certaine plage les déplacements relatifs latéraux du corps 12 par rapport à chaque plateau 4, voire on les interdit totalement.

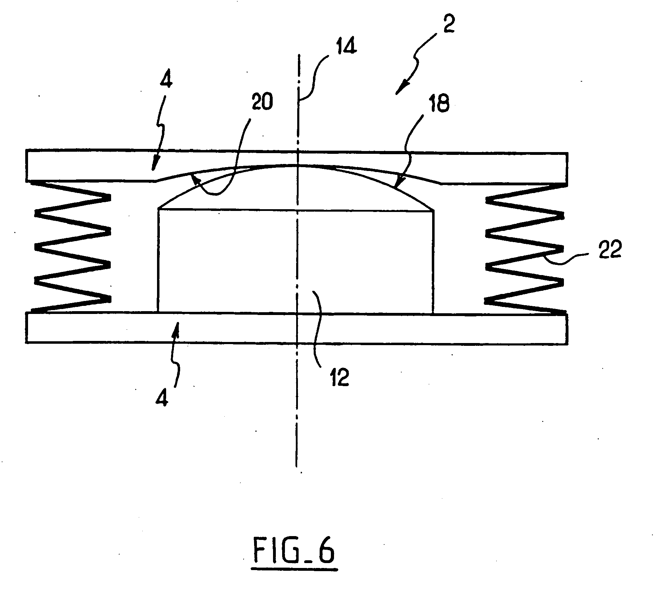

Dans la variante de la figure 6, la face 20 du plateau peut être courbe et concave dans une ou deux directions, comme c'est le cas ici, et la face 18 peut être courbe et convexe dans la ou les directions correspondantes, le rayon de courbure de la face 20 étant, pour chaque direction, plus grand que celui de la face 18 dans la direction correspondante. Les deux faces 18, 20 sont ici sphériques. Les rayons de courbure des surfaces 18 et 20 seront par exemple compris entre 70 et 80 mm, et 140 et 200 mm respectivement. Un tel agencement permet d'obtenir un auto-centrage des deux faces tout en autorisant un déplacement latéral relatif du corps 12 par rapport au plateau suivant une direction quelconque perpendiculaire à une direction longitudinale du rachis.

Dans le mode de réalisation de la figure 2, les deux extrémités du corps 12 présentent une surface de contact 18 avec le plateau associé de superficie variable et le rendant mobile latéralement par rapport au corps.

Au contraire, dans la variante de la figure 6, seule l'une des extrémités 18 du corps 12 présente cette propriété. L'autre extrémité, inférieure sur la figure 6, a une forme plane circulaire à zone de contact invariable avec le plateau associé et fixe par rapport à celui-ci.

Bien entendu, on pourra apporter à l'invention de nombreuses modifications sans sortir du cadre de celle-ci.

Le fluide pourra être un liquide, voire un mélange d'un liquide et d'un gaz, ce dernier étant par exemple faiblement soluble dans le liquide.

Le corps pourra avoir une forme elliptique en section transversale à l'axe 14.

La face interne 20 des plateaux 4 pourra être convexe, la face d'extrémité axiale 18 du corps 12 étant plane, ou concave à rayon de courbure plus grand que celui de la face 20 du plateau. Les deux faces en contact du plateau et du corps pourront être convexes.

La courbure des faces pourra être limitée à un seul plan.

On pourra mettre en oeuvre les caractéristiques relatives à l'enveloppe 22 (ressort, distance au corps 12) indépendamment des autres caractéristiques.

## Revendications

1. Prothèse de disque intervertébral comportant deux plateaux opposés (4), une partie intermédiaire (10) s'étendant entre les deux plateaux opposés (4) formant ainsi une enceinte fermée entre lesdits deux plateaux, ladite partie intermédiaire étant en outre adapté pour permettre à ces plateaux de bouger l'un par rapport à l'autre, un corps compressible (12) disposé dans ladite enceinte fermée, entre les deux plateaux (4), ledit corps compressible ayant une première surface (18) présentant une zone en contact avec une face (20) de l'un des plateaux dont la superficie augmente lorsqu'une force de compression est exercée sur ledit corps compressible, **caractérisée en ce que** ladite enceinte fermée comprend un fluide en contact avec lesdits plateaux, ledit fluide s'étendant en périphérie dudit corps compressible (12).

2. Prothèse selon le revendication 1, **caractérisée en ce que** ledit corps compressibles (12) posséde une seconde surface (18) opposée à la première surface, et cette première surface est en contact avec l'un des deux plateaux (4), tandis que la seconde surface est en contact avec l'autre des deux plateaux.

3. Prothèse selon la revendication 2, **caractérisée en ce que** ledit corps compressible (12) possède une hauteur définie entre les deux plateaux (4) qui diminue lorsque la force de compression est exercée sur l'un desdits plateaux.

4. Prothèse selon la revendication 1, **caractérisée en ce que** la première surface (18) dudit corps (12) est de forme convexe, possède une extrémité en contact avec l'un desdits plateaux (4), et la face (20) du plateau en contact avec la première surface dudit corps compressible est substantiellement plate.

5. Prothèse selon la revendication 1, **caractérisée en ce que** la première surface (18) dudit corps (12) est convexe, **en ce qu'**une face (20) du plateau en contact avec ladite première surface est concave, et **en ce que** la face concave possède un rayon de courbure qui est supérieur au rayon de courbure de ladite première surface convexe dudit corps compressible.

6. Prothèse selon la revendication 2, **caractérisée en ce que** la face (20) dudit plateau en contact avec ledit corps compressible (12) possède un renfoncement (32), et **en ce que** la première surface (18) dudit corps compressible est au moins disposée partiellement dans ce renfoncement.

7. Prothèse selon la revendication 6, **caractérisée en ce que** ledit renfoncement (32) est adapté pour limiter des déplacements relatifs latéraux du corps compressible (12) par rapport à chaque plateau (4).

8. Prothèse selon la revendication 1, **caractérisée en ce que** ledit corps compressible (12) comprend un matériau visco-élastique.

9. Prothèse selon la revendication 8, **caractérisée en ce que** ledit corps compressible (12) comprend du silicone.

10. Prothèse selon la revendication 1, **caractérisée en ce que** ledit fluide est compressible.

11. Prothèse selon la revendication 10, **caractérisée en ce que** ledit fluide est sélectionné dans le groupe consistant en : un liquide, un gaz, et un mélange de liquide et de gaz.

12. Prothèse selon la revendication 1, **caractérisée en ce que** lesdits plateaux (4) sont dimensionnés et profilés suivant la région lombaire de la colonne vertébrale.

13. Prothèse selon la revendication 1, **caractérisée en ce que** l'un des plateaux au moins comprend une plaque circulaire centrale (6), un anneaux (8) s'étendant autour d'une périphérie de la plaque circulaire centrale, au moins une gorge (27) s'étendant entre ladite plaque circulaire centrale et ledit anneau, et un joint (31) logé dans ladite gorge.

14. Prothèse selon la revendication 1, **caractérisée en ce que** ledit corps compressible (12) possède une dureté shore-A comprise entre 60 et 100, approximativement.

15. Prothèse selon la revendication 14, **caractérisée en ce que** ledit corps compressible (12) a une dureté shore-A d'environ 80 approximativement.

16. Prothèse selon la revendication 1, **caractérisée en ce que** ledit corps compressible (12) possède une première surface en contact avec l'un desdits plateaux (4); une seconde surface en contact avec l'autre desdits plateaux, et une paroi latérale sensiblement cylindrique s'étendant entre les première et seconde surfaces dudit corps compressible.

17. Prothèse selon la revendication 16, **caractérisée en ce que** les première et seconde surfaces dudit corps compressible (12) possèdent un profil sphérique convexe.

18. Prothèse selon la revendication 17, **caractérisée en ce que** les surfaces sphériques convexes desdites première et seconde surfaces possèdent un rayon de courbure sensiblement identique.

19. Prothèse selon la revendication 13, **caractérisée en ce que** ledit corps compressible (12) est en contact avec ladite plaque circulaire centrale (6).

20. Prothèse selon la revendication 13, **caractérisée en ce que** ledit corps compressible (12) possède un axe principal sensiblement aligné avec un point central de ladite plaque circulaire centrale (6).

21. Prothèse selon la revendication 1, **caractérisée en ce que** les deux plateaux (4) opposés s'étendent dans des plans sensiblement parallèles entre eux, et **en ce que** ledit corps compressible (12) est déplaçable dans des directions parallèles à ces plans.

22. Prothèse selon la revendication 1, **caractérisée en ce qu'**au moins l'un des plateaux (4) est apte à être fixé à un os.

23. Prothèse selon la revendication 22, **caractérisée en ce que** le plateau apte à être fixé à un os possède au moins une patte (25) faisant saillie vers l'extérieur et ayant un orifice (27) adapté pour recevoir une vis (26) destinée à être insérée à l'intérieur de l'os.

24. Prothèse selon la revendication 1, **caractérisée en ce que** ladite partie intermédiaire comprend un soufflet (22) ayant un bord supérieur fixé à l'un des plateaux et un bord inférieur fixé à l'autre des plateaux.

25. Prothèse selon la revendication 24, **caractérisée en ce que** le bord supérieur est fixé à la plaque circulaire centrale (6) de l'un des plateaux et le bord inférieur est fixé à la plaque circulaire centrale (6) de l'autre des plateaux.

26. Prothèse selon la revendication 25, **caractérisée en ce que** les bords supérieur et inférieur du soufflet (22) sont collés à un bord externe des plaques circulaires centrales (6), respectivement.

27. Prothèse selon la revendication 25, **caractérisée en ce que** ledit soufflet (22) possède une raideur d'environ 1,6 N/mm.

28. Prothèse selon la revendication 1, **caractérisée en ce que** lesdits plateaux (4) ont des surfaces externes recouvertes d'une substance pouvant stimuler la croissance osseuse.

29. Prothèse selon la revendication 28, **caractérisée en ce que** la substance comporte de l'hydroxyapatie.

30. Prothèse selon la revendication 1, **caractérisée en ce que** lesdits plateaux (4) possèdent une surface externe poreuse recouverte d'une substance pouvant stimuler la croissance osseuse.

31. Prothèse selon la revendication 1, **caractérisée en ce que** la seconde surface dudit corps compressible se trouvant à distance de la première surface est sensiblement plate et est en contact avec une partie plate de l'autre desdits plateaux (4).

32. Prothèse selon la revendication 1, **caractérisée en ce que** ledit corps compressible (12) est de forme elliptique, en section transversale.

33. Prothèse selon la revendication 1, **caractérisée en ce que** lesdits plateaux opposés (4) sont compressibles l'un par rapport à l'autre selon un axe principal de compression.

34. Prothèse selon la revendication 1, **caractérisée en ce que** ladite partie intermédiaire maintient lesdits plateaux opposés espacés d'une première distance l'un de l'autre lorsque aucune force de compression n'est exercée sur lesdits plateaux.

35. Prothèse selon la revendication 34, **caractérisée en ce que** lesdits plateaux opposés (4) sont compressibles jusqu'à une seconde distance d'espacement, cette distance étant inférieure à ladite première distance.

36. Prothèse selon la revendication 35, **caractérisée en ce que** la distance d'espacement entre les plateaux (4) est fonction des forces de compression exercées sur ces plateaux et suit une courbe ayant une forme en hystérésis.

## Claims

1. Intervertebral disc prosthesis comprising two opposite plates (4), an intermediate part (10) extending between the two opposite plates (4) and forming a closed chamber between said two plates, said intermediate part additionally being adapted to allow these plates to move relative to each other, a compressible body (12) disposed in said closed chamber, between the two plates (4), said compressible body having a first surface (18) with a zone in contact with a face (20) of one of the plates, the surface area of which increases when a compression force is exerted on said compressible body, **characterized in that** said closed chamber comprises a fluid in contact with said plates, said fluid extending around the periphery of said compressible body (12).

2. Prosthesis according to Claim 1**, characterized in that** said compressible body (12) has a second surface (18) opposite the first surface, and this first surface is in contact with one of the two plates (4), while the second surface is in contact with the other of the two plates.

3. Prosthesis according to Claim 2, **characterized in that** said compressible body (12) has a height defined between the two plates (4) that decreases when the compression force is exerted on one of said plates.

4. Prosthesis according to Claim 1**, characterized in that** the first surface (18) of said body (12) is of convex shape, has one end in contact with one of said plates (4), and the face (20) of the plate in contact with the first surface of said compressible body is substantially flat.

5. Prosthesis according to Claim 1**, characterized in that** the first surface (18) of said body (12) is convex, and **in that** a face (20) of the plate in contact with said first surface is concave, and **in that** the concave face has a radius of curvature greater than the radius of curvature of said convex first surface of said compressible body.

6. Prosthesis according to Claim 2**, characterized in that** the face (20) of said plate in contact with said compressible body (12) has a depression (32), and **in that** the first surface (18) of said compressible body is at least partially disposed in this depression.

7. Prosthesis according to Claim 6, **characterized in that** said depression (32) is adapted to limit relative lateral displacements of the compressible body (12) with respect to each plate (4).

8. Prosthesis according to Claim 1, **characterized in that** said compressible body (12) comprises a viscoelastic material.

9. Prosthesis according to Claim 8, **characterized in that** said compressible body (12) comprises silicone.

10. Prosthesis according to Claim 1, **characterized in that** said fluid is compressible.

11. Prosthesis according to Claim 10**, characterized in that** said fluid is selected from the group consisting of a liquid, a gas, and a mixture of liquid and gas.

12. Prosthesis according to Claim 1, **characterized in that** said plates (4) are dimensioned and profiled for the lumbar region of the vertebral column.

13. Prosthesis according to Claim 1, **characterized in that** at least one of the plates has a central circular dish (6), a ring (8) extending around a periphery of the central circular dish, at least one groove (27) extending between said central circular dish and said ring, and a seal (31) housed in said groove.

14. Prosthesis according to Claim 1, **characterized in that** said compressible body (12) has a Shore A hardness of between 60 and 100, approximately.

15. Prosthesis according to Claim 14, **characterized in that** said compressible body (12) has a Shore A hardness of approximately 80.

16. Prosthesis according to Claim 1**, characterized in that** said compressible body (12) has a first surface in contact with one of said plates (4), a second surface in contact with the other of said plates, and a substantially cylindrical side wall extending between the first and second surfaces of said compressible body.

17. Prosthesis according to Claim 16**, characterized in that** the first and second surfaces of said compressible body (12) have a convex spherical profile.

18. Prosthesis according to Claim 17, **characterized in that** the convex spherical surfaces of said first and second surfaces have a substantially identical radius of curvature.

19. Prosthesis according to Claim 13, **characterized in that** said compressible body (12) is in contact with said central circular dish (6).

20. Prosthesis according to Claim 13, **characterized in that** said compressible body (12) has a main axis substantially in alignment with a central point of said central circular dish (6).

21. Prosthesis according to Claim 1, **characterized in that** the two opposite plates (4) extend in planes that are substantially parallel to each other, and **in that** said compressible body (12) is displaceable in directions parallel to these planes.

22. Prosthesis according to Claim 1, **characterized in that** at least one of the plates (4) is able to be fixed to a bone.

23. Prosthesis according to Claim 22, **characterized in that** the plate able to be fixed to a bone has at least one lug (25) projecting outwards and having an orifice (27) adapted to receive a screw (26) that can be inserted into the bone.

24. Prosthesis according to Claim 1, **characterized in that** said intermediate part comprises a bellows (22) having an upper edge fixed to one of the plates and a lower edge fixed to the other of the plates.

25. Prosthesis according to Claim 24, **characterized in that** the upper edge is fixed to the central circular dish (6) of one of the plates, and the lower edge is fixed to the central circular dish (6) of the other of the plates.

26. Prosthesis according to Claim 25, **characterized in that** the upper and lower edges of the bellows (22) are bonded to a respective outer edge of the central circular dishes (6).

27. Prosthesis according to Claim 25, **characterized in that** said bellows (22) has a stiffness of approximately 1.6 N/mm.

28. Prosthesis according to Claim 1, **characterized in that** said plates (4) have outer surfaces covered by a substance that can stimulate bone growth.

29. Prosthesis according to Claim 28, **characterized in that** the substance includes hydroxyapatite.

30. Prosthesis according to Claim 1, **characterized in that** said plates (4) have a porous outer surface covered by a substance that can stimulate bone growth.

31. Prosthesis according to Claim 1, **characterized in that** the second surface of said compressible body remote from the first surface is substantially flat and is in contact with a flat part of the other of said plates (4).

32. Prosthesis according to Claim 1, **characterized in that** said compressible body (12) is elliptical in cross section.

33. Prosthesis according to Claim 1, **characterized in that** said opposite plates (4) are compressible towards each other along a main axis of compression.

34. Prosthesis according to Claim 1, **characterized in that** said intermediate part keeps said opposite plates spaced apart from each other by a first distance when no compression force is exerted on said plates.

35. Prosthesis according to Claim 34, **characterized in that** said opposite plates (4) are compressible to a second spacing distance, the latter distance being less than said first distance.

36. Prosthesis according to Claim 35, **characterized in that** the spacing distance between the plates (4) is a function of the compression forces exerted on these plates and follows a curve having a hysteresis form.

## Patentansprüche

1. Bandscheibenprothese, umfassend zwei gegenüberliegende Platten (4), ein Zwischenteil (10), das sich zwischen den beiden gegenüberliegen Platten (4) erstreckt und somit einen geschlossenen Raum zwischen den beiden Platten bildet, wobei das Zwischenteil ferner ausgelegt ist, um diesen Platten zu erlauben, sich im Verhältnis zueinander zu bewegen, einen kompressiblen Körper (12), der in dem geschlossenen Raum zwischen den beiden Platten (4) angeordnet ist, wobei der kompressible Körper eine erste Fläche (18) hat, die einen Bereich in Kontakt mit einer Seite (20) der einen der Platten aufweist, deren Oberfläche sich vergrößert, wenn eine Kompressionskraft auf den kompressiblen Körper ausgeübt wird, **dadurch gekennzeichnet, dass** der geschlossene Raum ein Fluid in Kontakt mit den Platten umfasst, wobei sich das Fluid in der Umgebung des kompressiblen Körpers (12) erstreckt.

2. Prothese gemäß Anspruch 1, **dadurch gekennzeichnet, dass** der kompressible Körper (12) eine zweite Fläche (18) gegenüber der ersten Fläche umfasst und diese erste Fläche in Kontakt mit der einen der beiden Platten (4) ist, während die zweite Fläche in Kontakt mit der anderen der beiden Platten ist.

3. Prothese gemäß Anspruch 2, **dadurch gekennzeichnet, dass** der kompressible Körper (12) eine Höhe besitzt, die zwischen den beiden Platten (4) festgelegt ist, die abnimmt, wenn die Kompressionskraft auf die eine der beiden Platten ausgeübt wird.

4. Prothese gemäß Anspruch 1, **dadurch gekennzeichnet, dass** die erste Fläche (18) des Körpers (12) von konvexer Form ist, ein Ende in Kontakt mit der einen der Platten (4) besitzt und die Seite (20) der Platte in Kontakt mit der ersten Fläche des kompressiblen Körpers im Wesentlichen flach ist.

5. Prothese gemäß Anspruch 1, **dadurch gekennzeichnet, dass** die erste Seite (18) des Körpers (12) konvex ist, dass eine Oberfläche (20) der Platte in Kontakt mit der ersten Fläche konkav ist und dass die konkave Seite einen Krümmungsradius besitzt, der größer als der Krümmungsradius der ersten konvexen Fläche des kompressiblen Körpers ist.

6. Prothese gemäß Anspruch 2, **dadurch gekennzeichnet, dass** die Seite (20) der Platte in Kontakt mit dem kompressiblen Körper (12) eine Verstärkung (32) besitzt und dass die erste Fläche (18) des kompressiblen Körpers wenigstens teilweise in dieser Verstärkung angeordnet ist.

7. Prothese gemäß Anspruch 6, **dadurch gekennzeichnet, dass** die Verstärkung (32) ausgelegt ist, um seitliche Relativverschiebungen des kompressiblen Körpers (12) in Bezug auf jede Platte (4) zu begrenzen.

8. Prothese gemäß Anspruch 1, **dadurch gekennzeichnet, dass** der kompressible Körper (12) ein viskoelastisches Material umfasst.

9. Prothese gemäß Anspruch 8, **dadurch gekennzeichnet, dass** der kompressible Körper (12) Silikon umfasst.

10. Prothese gemäß Anspruch 1, **dadurch gekennzeichnet, dass** das Fluid kompressibel ist.

11. Prothese gemäß Anspruch 10, **dadurch gekennzeichnet, dass** das Fluid aus der Gruppe ausgewählt, die besteht aus: eine Flüssigkeit, ein Gas, ein Mischung aus Flüssigkeit und Gas.

12. Prothese gemäß Anspruch 1, **dadurch gekennzeichnet, dass** die Platten (4) dem Lendenbereich der Wirbelsäule entsprechend dimensioniert und profiliert sind.

13. Prothese gemäß Anspruch 1, **dadurch gekennzeichnet, dass** die eine der Platten wenigstens eine zentrale kreisförmige Scheibe (6) umfasst, wobei sich ein Ring (8) um eine Umgebung der zentralen, kreisförmigen Scheibe erstreckt, wenigstens eine Rille (27), die sich zwischen der kreisförmigen, zentralen Scheibe und dem Ring erstreckt, sowie eine Dichtung (31), die in der Rille aufgenommen wird.

14. Prothese gemäß Anspruch 1, **dadurch gekennzeichnet, dass** der kompressible Körper (12) eine Shore-A Härte besitzt, die etwa zwischen 60 und 100 liegt.

15. Prothese gemäß Anspruch 14, **dadurch gekennzeichnet, dass** der kompressible Körper (12) etwa eine Shore-A Härte von ungefähr 80 hat.

16. Prothese gemäß Anspruch 1, **dadurch gekennzeichnet, dass** der kompressible Körper (12) eine erste Fläche in Kontakt mit der einen der Platten (4) besitzt, eine zweite Fläche in Kontakt mit der anderen der Platten und eine im Wesentlichen zylindrische Seitenwand, die sich zwischen der ersten und zweiten Fläche des kompressiblen Körpers erstreckt.

17. Prothese gemäß Anspruch 16, **dadurch gekennzeichnet, dass** die erste und zweite Fläche des kompressiblen Körpers (12) ein konvexes, kugelförmiges Profil besitzt.

18. Prothese gemäß Anspruch 17, **dadurch gekennzeichnet, dass** die kugelförmigen, konvexen Flächen der ersten und zweiten Fläche einen im Wesentlichen identischen Krümmungsradius besitzen.

19. Prothese gemäß Anspruch 13, **dadurch gekennzeichnet, dass** der kompressible Körper (12) in Kontakt mit der kreisförmigen, zentralen Scheibe (6) ist.

20. Prothese gemäß Anspruch 13, **dadurch gekennzeichnet, dass** der kompressible Körper (12) eine Hauptachse besitzt, die im Wesentlichen mit einem zentralen Punkt der kreisförmigen, zentralen Scheibe (6) ausgerichtet ist.

21. Prothese gemäß Anspruch 1, **dadurch gekennzeichnet, dass** die beiden gegenüberliegenden Platten (4) sich in im Wesentlichen parallelen Ebenen zwischen sich erstrecken und der kompressible Körper (12) in zu diesen Ebenen parallelen Ebenen verschiebbar ist.

22. Prothese gemäß Anspruch 1, **dadurch gekennzeichnet, dass** wenigstens die eine des Platten (4) geeignet ist, um an einem Knochen befestigt zu werden.

23. Prothese gemäß Anspruch 22, **dadurch gekennzeichnet, dass** die Platte, die geeignet ist, um an einem Knochen befestigt zu werden wenigstens eine Lasche (25) besitzt, die nach außen vorsteht und eine Öffnung (27) hat, die ausgelegt ist, um eine Schraube (26) aufzunehmen, die dazu bestimmt ist, in das Innere des Knochens eingeführt zu werden.

24. Prothese gemäß Anspruch 1, **dadurch gekennzeichnet, dass** das Zwischenteil einen Balg (22) umfasst, der einen oberen Rand hat, der an der einen der Platten befestigt ist, und einen unteren Rand, der an der anderen der Platten befestigt ist.

25. Prothese gemäß Anspruch 24, **dadurch gekennzeichnet, dass** der obere Rand an der kreisförmigen, zentralen Scheibe (6) der einen der Platten befestigt ist und der untere Rand an der kreisförmigen, zentralen Scheibe (6) der anderen der Platten befestigt ist.

26. Prothese gemäß Anspruch 25, **dadurch gekennzeichnet, dass** der obere und untere Rand des Balgs (22) jeweils an einen äußeren Rand der kreisförmigen, zentralen Scheiben (6) geklebt sind.

27. Prothese gemäß Anspruch 25, **dadurch gekennzeichnet, dass** der Balg (22) eine Federkonstante von ungefähr 1,6 N/mm besitzt.

28. Prothese gemäß Anspruch 1, **dadurch gekennzeichnet, dass** die Platten (4) äußere Flächen besitzen, die mit einer Substanz bedeckt sind, die das Knochenwachstum stimulieren kann.

29. Prothese gemäß Anspruch 28, **dadurch gekennzeichnet, dass** die Substanz Hydroxylapatit umfasst.

30. Prothese gemäß Anspruch 1, **dadurch gekennzeichnet, dass** die Platten (4) eine äußere, poröse Fläche besitzen, die mit einer Substanz bedeckt ist, die das Knochenwachstum stimulieren kann.

31. Prothese gemäß Anspruch 1, **dadurch gekennzeichnet, dass** die zweite Fläche des kompressiblen Körpers, die sich von der ersten Fläche beabstandet befindet, im Wesentlichen flach ist und in Kontakt mit einem flachen Teil der anderen der Platten (4) ist.

32. Prothese gemäß Anspruch 1, **dadurch gekennzeichnet, dass** der kompressible Körper (12) im Querschnitt von elliptischer Form ist.

33. Prothese gemäß Anspruch 1, **dadurch gekennzeichnet, dass** die gegenüberliegenden Platten (4) in Bezug zueinander entlang einer Hauptkompressionsachse kompressibel sind.

34. Prothese gemäß Anspruch 1, **dadurch gekennzeichnet, dass** das Zwischenteil die gegenüberliegenden Platten in einer ersten Distanz voneinander beabstandet hält, wenn keine Kompressionskraft auf die Platten ausgeübt wird.

35. Prothese gemäß Anspruch 34, **dadurch gekennzeichnet, dass** die gegenüberliegenden Platten (4) bis zu einer zweiten Beabstandungsdistanz kompressibel sind, wobei diese Distanz kleiner als die erste Distanz ist.

36. Prothese gemäß Anspruch 35, **dadurch gekennzeichnet, dass** die Beabstandungsdistanz zwischen den Platten (4) abhängig von den auf diese Platten ausgeübten Kompressionskräften ist und einer Krümmung folgt, die eine Hystereseform hat.
